# EUROPÄISCHE PATENTSCHRIFT

(11) **EP 3 328 341 B1**
(45) Veröffentlichungstag und Bekanntmachung des Hinweises auf die Patenterteilung: **13.12.2023**
(21) Anmeldenummer: 16741318.6
(22) Anmeldetag: 21.07.2016
(51) Int. Cl.: A61G 13/10, A47C 21/04, A61F 7/00

(54) **VORRICHTUNG ZUM ERWÄRMEN EINER PATIENTENLAGERFLÄCHE EINES OPERATIONSTISCHES**
DEVICE FOR HEATING A PATIENT BEARING AREA OF AN OPERATING TABLE
DISPOSITIF POUR CHAUFFER UNE SURFACE DE RÉCEPTION DE PATIENT D'UNE TABLE D'OPÉRATION

(30) Priorität: 30.07.2015 DE 102015112449
(43) Veröffentlichungstag der Anmeldung: 06.06.2018
(73) Patentinhaber: Maquet GmbH, 76437 Rastatt (DE)
(72) Erfinder: GAISER, Immanuel, 76599 Weisenbach (DE); KATZENSTEIN, Bernhard, 76473 Iffezheim (DE)
(74) Vertreter: Zacco GmbH
(86) Internationale Anmeldenummer: PCT/EP2016/067397
(87) Internationale Veröffentlichungsnummer: WO 2017/016973

(56) Entgegenhaltungen:
- WO-A2-01/95841
- WO-A2-2008/046110
- US-A1- 2010 011 502

## Beschreibung

Die Erfindung betrifft eine Vorrichtung zum Erwärmen einer Patientenlagerfläche eines Operationstisches mit einem Heizelement.

Bei Operationen ist es oftmals problematisch eine für den Patienten geeignete Körpertemperatur zu gewährleisten. Insbesondere bei Operationen, die Einschnitte im Brustraum, im Bauchraum oder an den Beinen erfordern, kann der Patient zur Wärmeisolierung aus Gründen des Infektionsschutzes nicht ausreichend abgedeckt werden, wodurch sich eine Hypothermie des Patienten ergeben kann. Darüber hinaus wird bei einigen Operationen, wie z. B. in der Herzchirurgie, eine künstliche Hypothermie des Patienten durch in dessen Temperaturhaushalt eingreifende Medikamente herbeigeführt.

Aus dem Dokument US 6,653,607 B2 ist ein in eine Matratze eingebettetes mit elektrischem Strom betriebenes Heizelement bekannt, das die von ihm erzeugte Wärme konduktiv auf die Matratze überträgt, wodurch dem auf der Matratze liegenden Patienten ebenfalls durch konduktive Wärmeübertragung Wärme zugeführt wird. Durch das Vorsehen einer von der Patientenlagerfläche separaten Matratze zum Erwärmen des Patienten erhöht sich der Aufwand zum Fixieren der Matratze auf der Patientenlagerfläche und des Patienten auf der Matratze. Zudem muss die Matratze zusätzlich zu der Patientenlagerfläche gereinigt und desinfiziert werden. Weiterhin zeichnen sich das Heizelement der Matratze, die nötigen Leitungsbahnen und Sensoren typischerweise beim Durchleuchten des Patienten mit Bildgebenden Verfahren auf dem Bild ab, wodurch die Diagnostik erschwert wird. Deshalb ist es sinnvoll, Materialien geringer Dichte zu verwenden, die schon bei geringer Strahlungsintensität röntgentransparent erscheinen.

Ferner können sich bei langen Operationen Probleme durch Dekubitus in den Bereichen des Patienten, die mit hohem Auflagedruck auf der Patientenlagerfläche aufliegen, ergeben. Die Probleme durch Dekubitus werden durch das Erwärmen der die Dekubitus-gefährdeten Bereiche des Patienten durch die Matratze weiter verschärft. Idealerweise sollte eine Wärmeübertragung auf den Patienten nur in Bereichen des Patienten, die mit geringem Auflagedruck auf einer Patientenlagerfläche aufliegen, erfolgen. Eine solche differenzierte Wärmeübertragung kann durch ein in die Matratze eingebettetes Heizelement nicht sichergestellt werden.

Weiterhin ist die Sicherung des Patienten vor Überhitzungen durch den ungleichmäßigen Auflagedruck des Patienten auf der Matratze bei einem elektrisch betriebenen Heizelement aufwendig. Hierzu ist eine zuverlässige Überwachung der Temperatur über die gesamte mögliche Auflagefläche hinweg erforderlich.

Ferner muss der Patient durch konstruktive Maßnahmen effektiv vor elektrischen Strömen geschützt werden.

Aus dem Dokument DE 20 2006 017 369 U1 ist eine Wärmedecke bekannt, in die warme Luft eingeleitet wird, die wiederum an der dem Patienten zugewandten Unterseite der Wärmedecke austritt und den Patienten erwärmt. Durch die austretende warme Luft wird zusätzlich zum Patienten auch die Umgebung erwärmt, sodass die Operateure bei einer Operation mit im warmen Luftstrom sind. Zudem ist das Risiko von Wundinfektionen des Patienten durch von der austretenden Luft aufgewirbelte oder mitgeführte Krankheitserreger erhöht.

Dokument US 2010/011502 A1 beschreibt ein klimatisiertes Bett, welches ein Heizelement, ein Fluidverteilungselement und ein oder mehrere Strömungsumlenkungselemente umfasst. Die Strömungsumlenkungselemente können dabei ein oder mehrere luftundurchlässige Materialien umfassen und dienen dem Zweck, ein Fluid, welches über die Kanäle in die eine Strömungsschicht geleitet werden soll, umzulenken, sodass es sich in translatorische Richtung verteilt.

Dokument WO 2008/046110 beschreibt eine Patientenlagerfläche eines Operationstisches mit einer Vorrichtung zum Erwärmen der Patientenlagerfläche, mit einem Heizelement zum Erwärmen eines zur Wärmeübertragung dienenden Fluids, einer elastisch verformbaren vom erwärmten Fluid durchströmten Strömungsschicht der Patientenlagerfläche, und mit einer fluidundurchlässigen Dichtungsschicht, die auf der Strömungsschicht angeordnet ist, wobei eine von der Strömungsschicht abgewandte Oberfläche der Dichtungsschicht eine zum Patientenkontakt vorgesehene Oberfläche der Patientenlagerfläche ist.

Aufgabe der Erfindung ist es, eine Patientenlagerfläche mit einer Vorrichtung zum Erwärmen der Patientenlagerfläche anzugeben, durch die dem Patienten auf schonende und einfache Weise Wärme zuführbar ist.

Diese Aufgabe wird durch eine Patientenlagerfläche eines Operationstischs mit den Merkmalen des Anspruchs 1 gelöst. Vorteilhafte Weiterbildungen der Erfindung sind in den abhängigen Ansprüchen angegeben.

Bei der Vorrichtung gemäß Anspruch 1 hat die Patientenlagerfläche eine elastisch verformbare vom erwärmten Fluid durchströmbare Strömungsschicht. Beim Durchströmen dieser Strömungsschicht mit dem erwärmten Fluid, kann dem Patienten einfach und schonend Wärme, insbesondere mindestens die Wärmemenge, die der Patient an die Umgebung abgibt, zugeführt werden. Überhitzungen einzelner Bereiche des Patienten können einfach vermieden werden, da die Temperatur des die Strömungsschicht der Patientenlagerfläche durchströmenden Fluids und/oder die erzeugte Strömung des Fluids durch die Strömungsschicht einfach einstellbar sind. Weder Fluid noch Strömungsschicht bilden eine in einem bildgebenden Verfahren störende Bildveränderung, sodass die Eignung der Patientenlagerfläche für eine Durchleuchtung des Patienten gegeben ist. Weiterhin wird durch die elastische Verformbarkeit der Strömungsschicht erreicht, dass sich der Volumenstrom des Fluids in den durch den Auflagedruck des Patienten stärker verformten Bereichen der Strömungsschicht reduziert im Vergleich zu den übrigen Bereichen und dadurch die Patientenlagerfläche in den stärker verformten Bereichen weniger erwärmt. Die Dekubitus-gefährdeten Bereiche des Patienten werden dadurch weniger erwärmt als die übrigen Bereiche, wodurch der Patient geschont wird. Die Elastizität der Strömungsschicht ermöglicht sowohl einen einfachen Aufbau der Patientenlagerfläche als auch eine bequeme Lagerung des Patienten. Insbesondere kann auf eine weitere elastische Schicht zum druckentlastenden Lagern des Patienten zwischen der Strömungsschicht und der Oberfläche der Patientenlagerfläche verzichtet werden. Durch die Integration der Strömungsschicht in die Patientenlagerfläche sind keine zusätzlichen Auflagen oder Decken zu handhaben oder zu reinigen.

Das Fluid überträgt beim Durchströmen der Strömungsschicht Wärme auf die Patientenlagerfläche, die ihrerseits über ihre Oberfläche konduktiv Wärme auf den Patienten überträgt. Das zum Durchströmen der Strömungsschicht vorgesehene Fluid ist vorzugsweise ein Gas, wie z. B. Luft, oder eine Flüssigkeit. Die Strömungsschicht ist vorzugsweise ein offenporiger Schaumstoff, ein Vlies, eine flache aufblasbare Struktur und/oder eine dreidimensionale Textilstruktur. Bei einer elastischen Verformung wird die die Querschnittsfläche der Strömungsschicht kleiner, sodass sich der Strömungsquerschnitt verkleinert. Besonders vorteilhaft ist es, wenn die Strömungsschicht durch einen offenporigen Schaumstoff gebildet ist. Dadurch wird gewährleistet, dass die Strömungsschicht gleichzeitig durchströmbar und elastisch verformbar ist. Vorzugsweise ist als Schaumstoff Polyurethan vorgesehen.

Die Strömungsschicht hat mindestens einen Eintrittsbereich zum Einleiten des von dem Heizelement erwärmten Fluids in die Strömungsschicht und mindestens einen Austrittsbereich zum Austreten des Fluids aus der Strömungsschicht.

Dadurch kann das Heizelement außerhalb der Strömungsschicht vorgesehen werden und beeinträchtigt nicht deren elastische Verformbarkeit.

In einer besonders vorteilhaften Ausführungsform ist ein Strömungserzeuger vorgesehen, mit dessen Hilfe ein Fluidstrom zum Einleiten des Fluids in den Eintrittsbereich der Strömungsschicht, zum Durchströmen der Strömungsschicht und zum Austreten aus dem Austrittsbereich der Strömungsschicht erzeugbar ist. Dadurch wird eine effektive Umwälzung des Fluids und eine möglichst gleichmäßige Übertragung der Wärme von dem Fluid auf die Strömungsschicht erreicht. Wenn Luft als Fluid vorgesehen ist, ist der Strömungserzeuger vorzugsweise ein Ventilator oder ein Gebläse. Wenn eine Flüssigkeit als Fluid vorgesehenen ist, wird als Strömungserzeuger vorzugsweise eine Pumpe verwendet.

Ferner ist es vorteilhaft, wenn die Patientenlagerfläche mindestens ein Lagerflächensegment mit einem Polster hat. Dabei ist die Oberfläche des Lagerflächensegments eine Oberfläche des Polsters des Lagerflächensegments. Weiterhin sind die Strömungsschicht und eine Dichtungsschicht Bestandteil des Polsters des Lagerflächensegments. Dadurch ist ein modularer Aufbau der Patientenlagerfläche mit mehreren Lagerflächensegmenten möglich, von denen das Polster eines oder mehrerer Lagerflächensegmente erwärmbar ist.

In einer besonders vorteilhaften Ausgestaltung ist die zum Patientenkontakt vorgesehene Oberfläche der Patientenlagerfläche eine Oberfläche der Dichtungsschicht. Dadurch wird eine schnelle und effektive Übertragung der Wärme von der Strömungsschicht auf den Patienten gewährleistet. Als Dichtungsschicht ist vorzugsweise eine sogenannte Polyurethansprühhaut vorgesehen.

Ferner ist es vorteilhaft, wenn eine an die Strömungsschicht angrenzende Wärmespeicherschicht zum Speichern der Wärme des Fluids vorgesehen ist. Dadurch wird erreicht, dass von der Strömungsschicht auf nicht den Patienten kontaktierende Bereiche der Patientenlagerfläche übertragene Wärme gespeichert wird und eine längere Übertragungsdauer von Wärme an den Patienten nach einem Abschalten des Heizelements ermöglicht wird. Vorzugsweise werden für die Wärmespeicherschicht Latentwärmespeicher auf Wachsbasis oder auf der Basis übersättigter Lösungen und generell Materialien mit hoher Wärmekapazität, wie z. B. Silikongele, Polyurethangele, Wassermatten oder Ölmatten, vorgesehen. Die Wärmespeicherschicht kann dabei sowohl in der Strömungsschicht als auch zwischen dem Patienten und der Strömungsschicht, d.h. oberhalb der Strömungsschicht oder unterhalb der Strömungsschicht angeordnet sein.

Besonders vorteilhaft ist es, wenn der mindestens eine Eintrittsbereich und der mindestens eine Austrittsbereich außerhalb eines für die Durchleuchtung des Patienten mit bildgebenden Verfahren vorgesehenen Bereichs der Patientenlagerfläche angeordnet sind. Dadurch wird vermieden, dass der Eintrittsbereich oder der Austrittsbereich auf einem bei der Durchleuchtung des Patienten erzeugten Bild stören.

In einer weiteren vorteilhaften Ausgestaltung ist das Heizelement in der Patientenlagerfläche angeordnet. Dadurch wird eine kompakte Anordnung der Vorrichtung erreicht und das Heizelement durch die Oberfläche der Patientenlagerfläche vor Verunreinigungen geschützt. Die Wärmeübertragung vom Heizelement auf das Fluid erfolgt vorzugsweise über einen mit dem Heizelement verbundenen Wärmetauscher.

Ferner ist es vorteilhaft, wenn das Heizelement ein röntgentransparentes Flächenheizelement ist und das Fluid bei aktiviertem Strömungserzeuger an der Heizfläche des Flächenheizelements vorbeiströmt. Dadurch wird eine effektive Wärmeübertragung auf das Fluid erreicht. Alternativ oder zusätzlich kann das Heizelement in einem Randbereich der Patientenlagerfläche, insbesondere in einem Randbereich eines Polsters der Patientenlagerfläche, angeordnet sein. Ein Patient wird auf der Patientenlagerfläche vorzugsweise so positioniert, dass er nicht im Randbereich aufliegt, zumindest dass in dem Randbereich keine relevanten zu durchleuchtenden Bereiche des Patienten positioniert sind.

Besonders vorteilhaft ist es, wenn der Eintrittsbereich an einem ersten Ende des Lagerflächensegments und der Austrittsbereich an einem dem ersten Ende in Längsrichtung der Patientenlagerfläche gegenüberliegenden zweiten Ende des Lagerflächensegments angeordnet sind. Dadurch wird eine gute Durchströmung der Strömungsschicht erreicht, die bei einer Durchströmung quer zur Längsrichtung der Patientenlagerfläche durch eine elastische Verformung der Strömungsschicht entlang der Längsachse der Patientenlagerfläche eingeschränkt sein kann.

Ferner ist es vorteilhaft, wenn das Lagerflächensegment einen weiteren Eintrittsbereich zum Einleiten des von dem Heizelement erwärmten Fluids in die Strömungsschicht hat und wenn das Lagerflächensegment einen weiteren Austrittsbereich zum Austreten des Fluids aus der Strömungsschicht hat. Dadurch kann eine gleichmäßige Erwärmung des Lagerflächensegments erreicht werden.

In einer vorteilhaften Ausführungsform der vorstehend erläuterten Ausgestaltung sind eine Steuereinheit und ein Ventilsystem zum Steuern des Fluidstroms durch die Strömungsschicht vorgesehen. Dabei ist das Ventilsystem derart durch die Steuereinheit steuerbar, dass das Fluid nur in einem Eintrittsbereich in die Strömungsschicht eingeleitet wird. Dadurch wird eine zeitlich gesteuerte Abfolge unterschiedlicher Strömungsmuster durch die Strömungsschicht ermöglicht, sodass das Lagerflächensegment flexibel erwärmbar ist.

Ferner ist es vorteilhaft, wenn eine Reinigungseinheit zum Reinigen von kontaminiertem Fluid vorgesehen ist. Dadurch wird eine Ansammlung von Krankheitserregern in dem Fluid vermieden, die unter Umständen in den Körper des Patienten gelangen könnten. Die Reinigungseinheit ist vorzugsweise geeignet die Krankheitserreger aus dem Fluid zu entfernen und/oder zu inaktivieren. Vorzugsweise ionisiert und/oder filtert die Reinigungseinheit das Fluid.

In einer besonders vorteilhaften Ausgestaltung ist das Lagerflächensegment ein erstes Lagerflächensegment, wobei das erste Lagerflächensegment einen ersten Anschluss zum Einleiten des Fluids in das erste Lagerflächensegment und einen zweiten Anschluss zum Austreten des Fluids aus dem ersten Lagerflächensegment hat. Weiterhin hat die Patientenlagerfläche mindestens ein zweites Lagerflächensegment mit einem ersten Anschluss zum Einleiten des Fluids in das zweite Lagerflächensegment und einen zweiten Anschluss zum Austreten des Fluids aus dem zweiten Lagerflächensegment. Des Weiteren hat auch das zweite Lagerflächensegment eine elastisch verformbare von erwärmtem Fluid durchströmbare Strömungsschicht und eine zwischen einer zum Patientenkontakt vorgesehenen Oberfläche des zweiten Lagerflächensegments und der Strömungsschicht des zweiten Lagerflächensegments angeordnete fluidundurchlässige Dichtungsschicht. Ferner hat die Strömungsschicht des zweiten Lagerflächensegments mindestens einen Eintrittsbereich zum Einleiten des von dem Heizelement erwärmten Fluids in die Strömungsschicht des zweiten Lagerflächensegments und mindestens einen Austrittsbereich zum Austreten des Fluids aus der Strömungsschicht des zweiten Lagerflächensegments. Dabei ist der zweite Anschluss des ersten Lagerflächensegments mit dem ersten Anschluss des zweiten Lagerflächensegments derart verbunden, dass das Fluid bei aktiviertem Strömungserzeuger durch den ersten Anschluss des ersten Lagerflächensegments in das erste Lagerflächensegment eingeleitet wird, von dem ersten Lagerflächensegment in das zweite Lagerflächensegment strömt, in den Eintrittsbereich der Strömungsschicht des zweiten Lagerflächensegments eingeleitet wird, aus dem Austrittsbereich der Strömungsschicht des zweiten Lagerflächensegments austritt und aus dem zweiten Anschluss des zweiten Lagerflächensegments austritt. Dadurch wird ein Durchleiten des Fluids von dem ersten Lagerflächensegment in das zweite Lagerflächensegment erreicht, wodurch nur ein Heizelement für die Erwärmung mehrerer Lagerflächensegmente vorgesehen werden muss. Die Durchleitung des Fluids kann entsprechend auf ein drittes Lagerflächensegment oder weitere Lagerflächensegmente ausgedehnt werden. Dabei werden vorzugsweise ein sternförmiger Aufbau, ein ringförmiger Aufbau oder Stichleitungen verwendet.

Besonders vorteilhaft ist es, wenn ein geschlossener Fluidkreislauf vorgesehen ist, in dem die Strömungsschicht, das Heizelement und der Strömungserzeuger angeordnet sind. Durch das Vorsehen eines solchen geschlossenen Systems wird das Eindringen von Krankheitserregern in das Fluid oder andere Bereiche der Vorrichtung erschwert. Der geschlossene Fluidkreislauf ist dabei nach außen fluiddicht.

Ferner ist es vorteilhaft, wenn Luft als Fluid vorgesehen ist und Luft aus der Umgebung mit Hilfe des Strömungserzeugers ansaugbar ist. Dabei wird die bei aktiviertem Strömungserzeuger angesaugte Luft aus der Umgebung in den Eintrittsbereich der Strömungsschicht des Lagerflächensegments eingeleitet. Dadurch werden ein besonders einfacher Aufbau und eine kostengünstige Fertigung der Vorrichtung erreicht. Die in dem Eintrittsbereich der Strömungsschicht eingeleitete Luft tritt nach einer Erwärmung der Strömungsschicht aus dieser und aus dem Lagerflächensegment aus. Das sich durch diesen Aufbau ergebende System hat einen offenen Kreislauf.

Weitere Merkmale und Vorteile der Erfindung ergeben sich aus der nachfolgenden Beschreibung, die die Erfindung anhand von Ausführungsbeispielen im Zusammenhang mit den beigefügten Figuren näher erläutert.

Es zeigen:
- Figur 1: eine schematische perspektivische Ansicht einer ersten Patientenlagerfläche;
- Figur 2: eine perspektivische Darstellung eines Lagerflächensegments einer Vorrichtung zum Erwärmen der Patientenlagerfläche gemäß einer ersten Ausführungsform;
- Figur 3: eine perspektivische Ansicht des Lagerflächensegments, wobei eine Polstereinheit und eine Trägerplatte des Lagerflächensegments zu Illustrationszwecken in einem unverbundenen Zustand dargestellt sind, sodass in dem Lagerflächensegment angeordnete Elemente sichtbar sind;
- Figur 4: eine perspektivische teilgeschnittene Darstellung des Lagerflächensegments;
- Figur 5: eine vergrößerte Darstellung eines in Figur 4 gekennzeichneten Bereichs A;
- Figur 6: einen Querschnitt des Lagerflächensegments entlang einer senkrechten Schnittebene quer zur Patientenlagerfläche;
- Figur 7: eine vergrößerte Darstellung eines in Figur 6 gekennzeichneten Bereichs B des Lagerflächensegments;
- Figur 8: einen Querschnitt eines Lagerflächensegments einer Vorrichtung zum Erwärmen des Lagerflächensegments gemäß einer zweiten Ausführungsform;
- Figur 9: eine vergrößerte Darstellung eines in Figur 8 gekennzeichneten Bereichs C des Lagerflächensegments;
- Figur 10: eine perspektivische Darstellung eines vergrößerten teilgeschnittenen Ausschnitts des Lagerflächensegments gemäß der zweiten Ausführungsform;
- Figur 11: einen Querschnitt eines Lagerflächensegments einer Vorrichtung zum Erwärmen des Lagerflächensegments gemäß einer dritten Ausführungsform;
- Figur 12: eine vergrößerten Darstellung eines in Figur 11 gekennzeichneten Bereichs D des Lagerflächensegments gemäß der dritten Ausführungsform;
- Figur 13: eine perspektivische Darstellung eines vergrößerten teilgeschnittenen Ausschnitts des Lagerflächensegments gemäß der dritten Ausführungsform;
- Figur 14: eine Draufsicht eines Lagerflächensegments einer Vorrichtung gemäß einer vierten Ausführungsform;
- Figur 15: eine Ansicht der Unterseite des Lagerflächensegments gemäß der vierten Ausführungsform;
- Figur 16: eine schematische perspektivische Darstellung eines Querschnitts des Lagerflächensegments und einer externen Wärmeerzeugungsund Umwälzeinheit gemäß der vierten Ausführungsform;
- Figur 17: eine Draufsicht einer schematisch dargestellten Patientenlagerfläche, die ein quer zur Längsachse der Patientenlagerfläche durchströmtes Lagerflächensegment hat;
- Figur 18: eine Draufsicht einer weiteren schematisch dargestellten Patientenlagerfläche, die ein von Luft in Richtung der Längsachse der Patientenlagerfläche durchströmtes Lagerflächensegment hat;
- Figur 19: eine Draufsicht einer weiteren schematisch dargestellten Patientenlagerfläche, in einer ersten Konfiguration zum Durchströmen eines Lagerflächensegments;
- Figur 20: eine Draufsicht der Patientenlagerfläche nach Figur 19 in einer zweiten Konfiguration zum Durchströmen eines Lagerflächensegments;
- Figur 21: eine Draufsicht der Patientenlagerfläche nach den Figuren 19 und 20 in einer dritten Konfiguration zum Durchströmen eines Lagerflächensegments; und
- Figur 22: eine schematische perspektivische Ansicht einer Vorrichtung zum Erwärmen von zwei sequentiell von einem Fluid durchströmbaren Lagerflächensegmenten gemäß einer weiteren Ausführungsform.

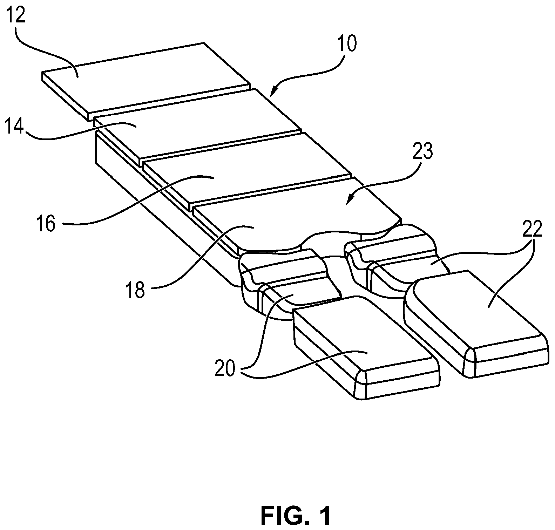

Figur 1 zeigt eine schematische perspektivische Ansicht einer Patientenlagerfläche 10 gemäß einer ersten Ausführungsform. Die Patientenlagerfläche 10 hat mehrere in ihrer Lage zueinander verstellbare Lagerflächensegmente, die eine unterschiedliche Positionierung eines nicht dargestellten Patienten ermöglichen. Im vorliegenden Ausführungsbeispiel umfassen die Lagerflächensegmente der Patientenlagerfläche 10 eine Kopfplatte 12, eine Rückenplatte 14, eine Rumpfplatte 16, eine Beckenplatte 18, eine zweiteilige rechte Beinplatte 20 und eine zweiteilige linke Beinplatte 22, von denen beispielhaft die Beckenplatte 18 als ein erfindungsgemäßes Lagerflächensegment einer Vorrichtung 23 zum Erwärmen der Patientenlagerfläche 10 ausgebildet ist. Bei anderen Ausführungsformen kann eine erfindungsgemäße Vorrichtung 23 zum Erwärmen der Patientenlagerfläche 10 weitere Lagerflächensegmente in gleicher Weise, alternativ oder zusätzlich wie die Beckenplatte 18 umfassen.

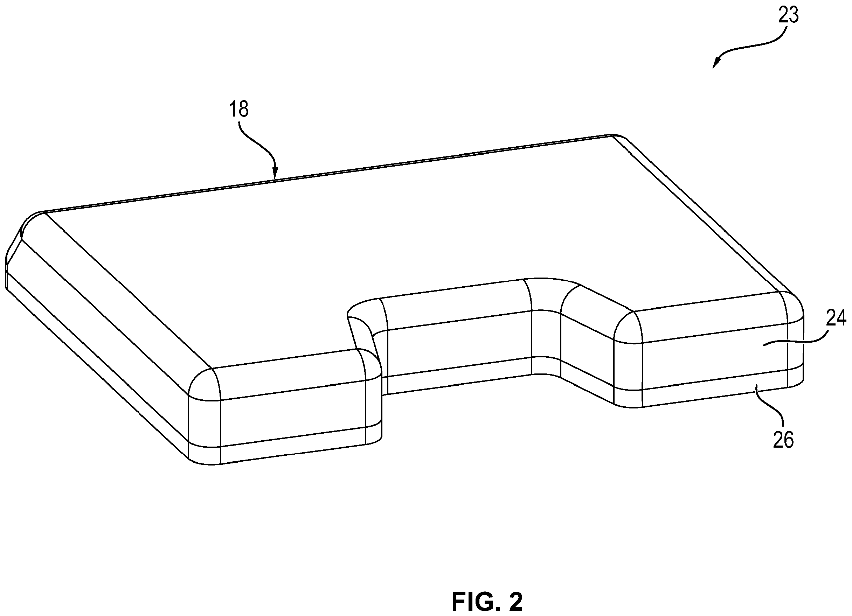

In Figur 2 ist eine perspektivische Darstellung der Beckenplatte 18 gemäß einer ersten Ausführungsform gezeigt. Die Beckenplatte 18 umfasst eine Polstereinheit 24 und eine mit dieser verbundenen Trägerplatte 26. Elemente mit gleichem Aufbau oder gleicher Funktion haben dieselben Bezugszeichen.

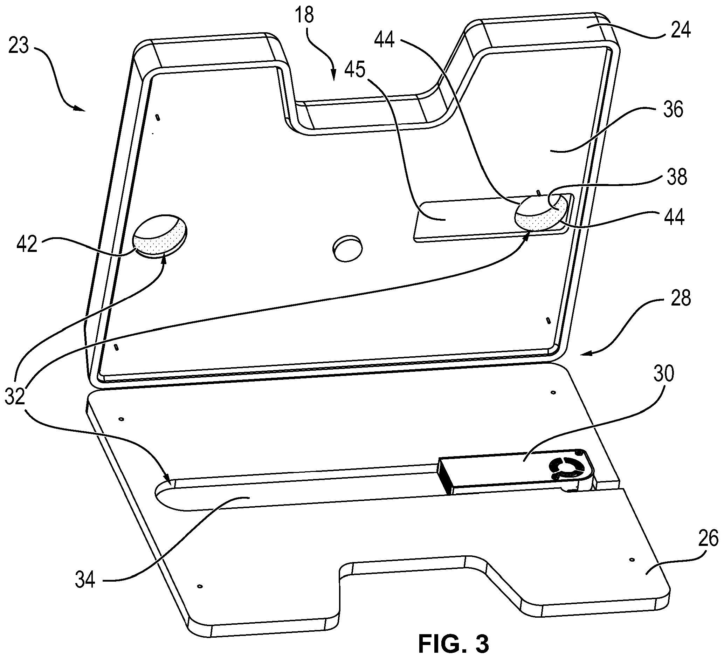

Figur 3 zeigt eine perspektivische Ansicht der Beckenplatte 18, wobei die Polstereinheit 24 und die Trägerplatte 26 zu Illustrationszwecken in einem unverbundenen Zustand dargestellt sind, sodass in der Beckenplatte 18 angeordnete Elemente sichtbar sind. Die Beckenplatte 18 umfasst eine Wärmeerzeugungs- und Umwälzeinheit 30 und ein in der Trägerplatte 26 und der Polstereinheit 24 ausgebildetes von erwärmter Luft durchströmbares Strömungskanalsystem 32.

Ein erster Strömungskanal des Strömungskanalsystems 32 ist durch eine Aussparung 34 in der Trägerplatte 26 gebildet. Die Trägerplatte 26 wird in dem in Figur 2 gezeigten verbundenen Zustand von einer in Figur 3 sichtbaren inneren Trägerplatte 36 der Polstereinheit 24 kontaktiert. An der von der Trägerplatte 26 abgewandten Seite der inneren Trägerplatte 36 ist eine elastisch verformbare, luftundurchlässige Polsterschicht 38 der Polstereinheit 24 angeordnet, deren Luftdurchlässigkeit wesentlich geringer ist als die der luftdurchlässigen Strömungsschicht 46.

Die Polsterschicht 38 und die innere Trägerplatte 36 haben eine Zuleitungsöffnung 42 und eine Austrittsöffnung 44, die jeweils als ein gemeinsames Durchgangsloch in der Polsterschicht 38 und der inneren Trägerplatte 36 ausgebildet sind. Die innere Trägerplatte 36 hat eine Aussparung 45, in die die in der Aussparung 34 der Trägerplatte 26 angeordnete Wärmeerzeugungs- und Umwälzeinheit 30 hineinragt, wenn die Trägerplatte 26 und die innere Trägerplatte 36 aneinander anliegen.

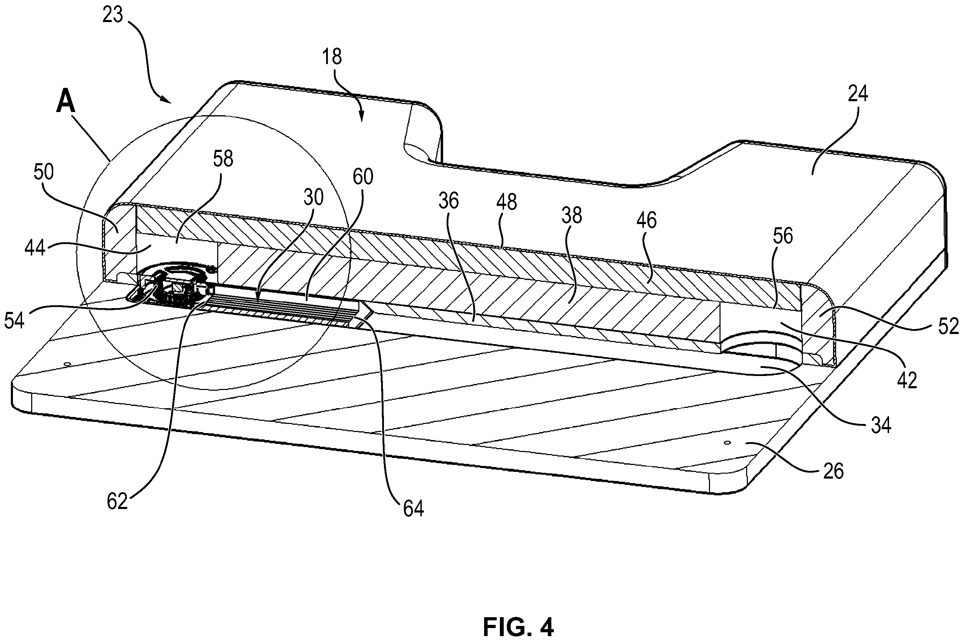

Figur 4 zeigt eine perspektivische teilgeschnittene Darstellung der Beckenplatte 18. Die Polstereinheit 24 umfasst die innere Trägerplatte 36, die Polsterschicht 38, die Zuleitungsöffnung 42, eine luftdurchlässige Strömungsschicht 46, eine luftundurchlässige Dichtungsschicht 48, ein erstes luftundurchlässiges Seitenpolster 50, ein zweites luftundurchlässiges Seitenpolster 52 und die Austrittsöffnung 44. Dabei bildet die Zuleitungsöffnung 42 einen zweiten Strömungskanal des Strömungskanalsystems 32, die Strömungsschicht 46 einen dritten Strömungskanal des Strömungskanalsystems 32 und die Austrittsöffnung 44 einen vierten Strömungskanal des Strömungssystems 32.

Die Strömungsschicht 46 hat einen Eintrittsbereich 56, der an einem ersten Ende der Zuleitungsöffnung 42 angeordnet ist. Das dem ersten Ende der Zuleitungsöffnung 42 gegenüberliegende zweite Ende grenzt an die Aussparung 34 der Trägerplatte 26, in der die Wärmeerzeugungs- und Umwälzeinheit 30 angeordnet ist. Die Wärmeerzeugungs- und Umwälzeinheit 30 hat eine Umwälzeinheit 54 und ein Heizelement 60, das eine Lufteintrittsöffnung 62 und eine Luftaustrittsöffnung 64 hat. Die Umwälzeinheit 54 saugt Luft über die Austrittsöffnung 44 einen Austrittsbereich 58 der Strömungsschicht 46 an und führt sie dem Heizelement 60 zu, das diese dann erwärmt.

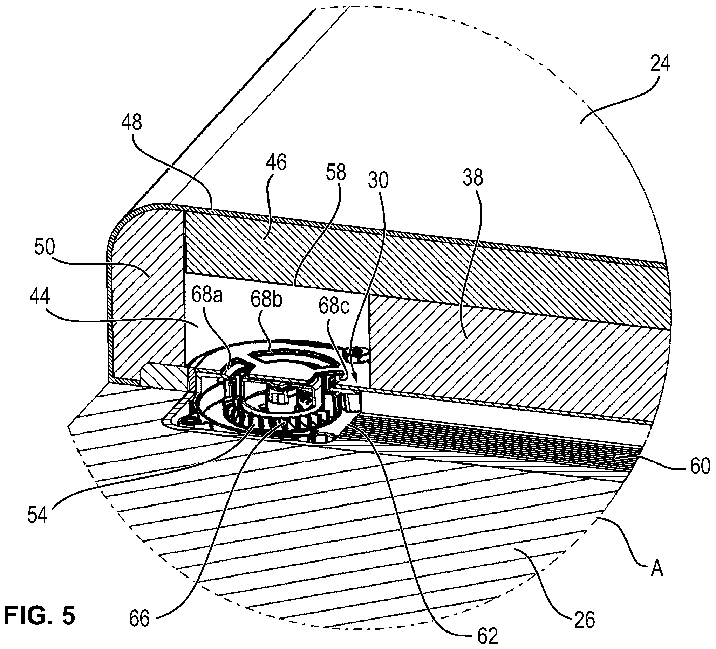

Figur 5 zeigt eine vergrößerte Darstellung eines in Figur 4 gekennzeichneten Bereichs A, der die Wärmeerzeugungs- und Umwälzeinheit 30 umfasst. Die Umwälzeinheit 54 hat in Richtung der Austrittsöffnung 44 ausgerichtete Eintrittsöffnungen 68a, 68b und 68c und einen Radialventilator 66, der Luft mittig aus den Eintrittsöffnungen 68a, 68b und 68c ansaugt und über die Lufteintrittsöffnung 62 zum Heizelement 60 weiterleitet.

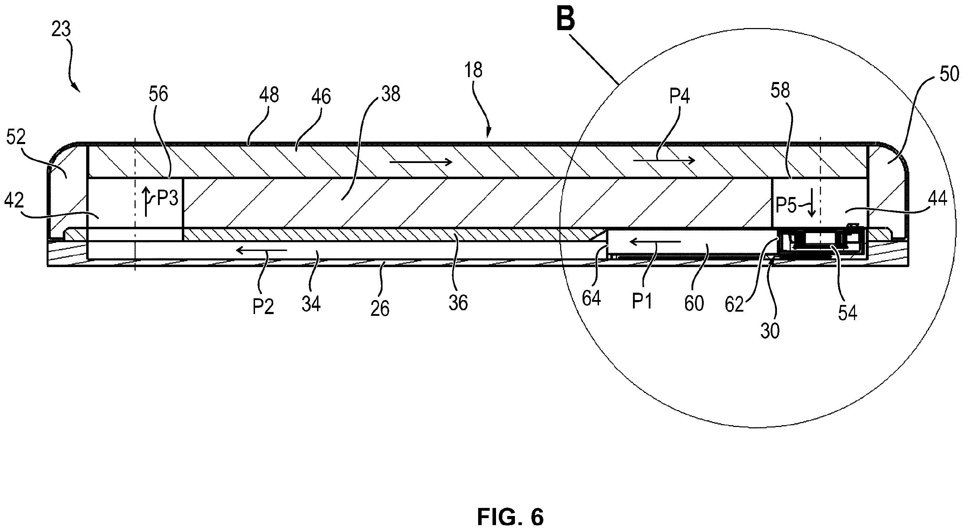

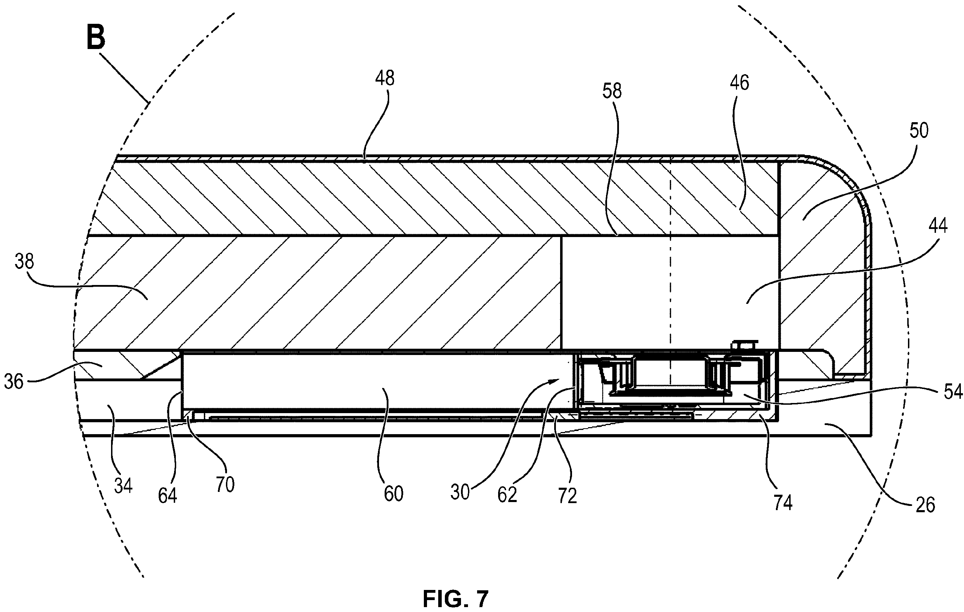

Figur 6 zeigt einen Querschnitt der Beckenplatte 18 entlang einer senkrechten Schnittebene quer zur Patientenlagerfläche 10, von dem in Figur 7 ein in Figur 6 mit B gekennzeichneter Bereich in einer vergrößerten Darstellung gezeigt wird. Die Umwälzeinheit 54 erzeugt in dem Strömungskanalsystem 32 der Beckenplatte 18 einen Luftstrom, dessen Richtung in den vier Strömungskanälen und in der Wärmeerzeugungs- und Umwälzeinheit 30 jeweils durch in Figur 6 eingezeichnete Pfeile P1 bis P5 dargestellt ist.

Bei aktiviertem Radialventilator 66 saugt die Umwälzeinheit 54 Luft aus der Austrittsöffnung 44 an und führt diese dem Heizelement 60 über dessen Lufteintrittsöffnung 62 zu, sodass die Luft an einem Wärmetauscher des Heizelements 60 in Richtung des Pfeils P1 vorbeiströmt und über die Luftaustrittsöffnung 64 in den durch die Aussparung 34 gebildeten ersten Strömungskanal austritt. Im ersten Strömungskanal strömt die Luft in Richtung des Pfeils P2 in die Zuleitungsöffnung 42, in dieser entlang des Pfeils P3 über den Eintrittsbereich 56 in die Strömungsschicht 46. In der Strömungsschicht 46 strömt die Luft in Richtung des Pfeils P4 und erwärmt dabei die Strömungsschicht 46 und die Dichtungsschicht 48. Nach dem Durchströmen der Strömungsschicht 46 strömt die abgekühlte Luft über den Austrittsbereich 58 aus der Strömungsschicht 46 in die Austrittsöffnung 44 in Richtung des Pfeils P5.

Anschließend wird die Luft wieder von der Umwälzeinheit 54 angesaugt, sodass sie einen durch die Strömungskanäle gebildeten geschlossenen Strömungskreislauf einmal durchlaufen hat. Bei anderen Ausführungsformen kann die Luft auch in der den Pfeilen P1 bis P5 entgegengesetzten Richtung strömen, wenn eine andere entsprechend ausgebildete Umwälzeinheit mit umgekehrter Strömungsrichtung verwendet wird.

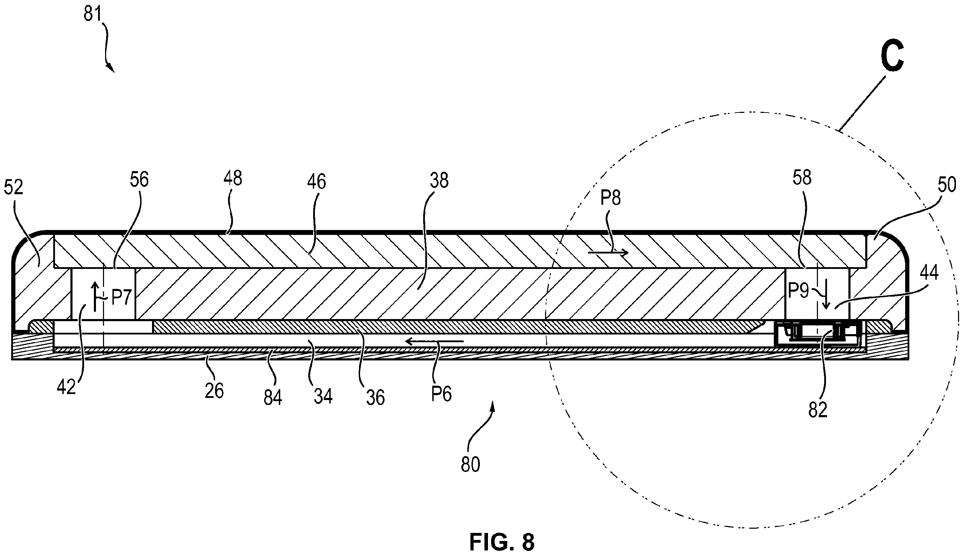

Figur 8 zeigt einen Querschnitt eines Lagerflächensegments 80 einer Vorrichtung 81 zum Erwärmen des Lagerflächensegments 80 gemäß einer zweiten Ausführungsform. Das Lagerflächensegment 80 hat einen ähnlichen Aufbau wie die Beckenplatte 18 der ersten Ausführungsform. Elemente mit dem gleichen Aufbau oder der gleichen Funktion haben dieselben Bezugszeichen. Anstelle des Wärmeerzeugungs- und Umwälzelements 30 der Beckenplatte 18 der ersten Ausführungsform sind in dem Lagerflächensegment 80 der zweiten Ausführungsform eine Umwälzeinheit 82 und ein Flächenheizelement 84 vorgesehen.

Die in Figur 8 eingezeichneten Pfeile P6 bis P9 geben die Richtung des Luftstroms bei aktivierter Umwälzeinheit 82 an. Dabei wird der Luftstrom beim Durchströmen der Aussparung 34 durch das Flächenheizelement 84 erwärmt. Die erwärmte Luft tritt anschließend in die Zuleitungsöffnung 42 ein.

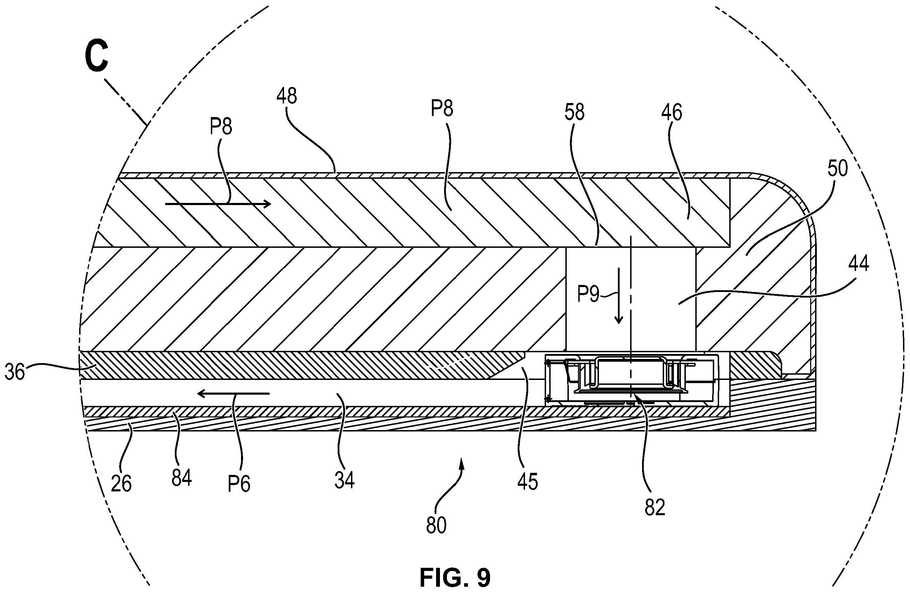

Wie in Figur 9 in einer vergrößerten Darstellung eines in Figur 8 mit C gekennzeichneten Bereichs des Lagerflächensegments 80 gezeigt ist, kontaktiert die Umwälzeinheit 82 das Flächenheizelement 84 und die Polsterschicht 38, sodass keine Dichtungen erforderlich sind.

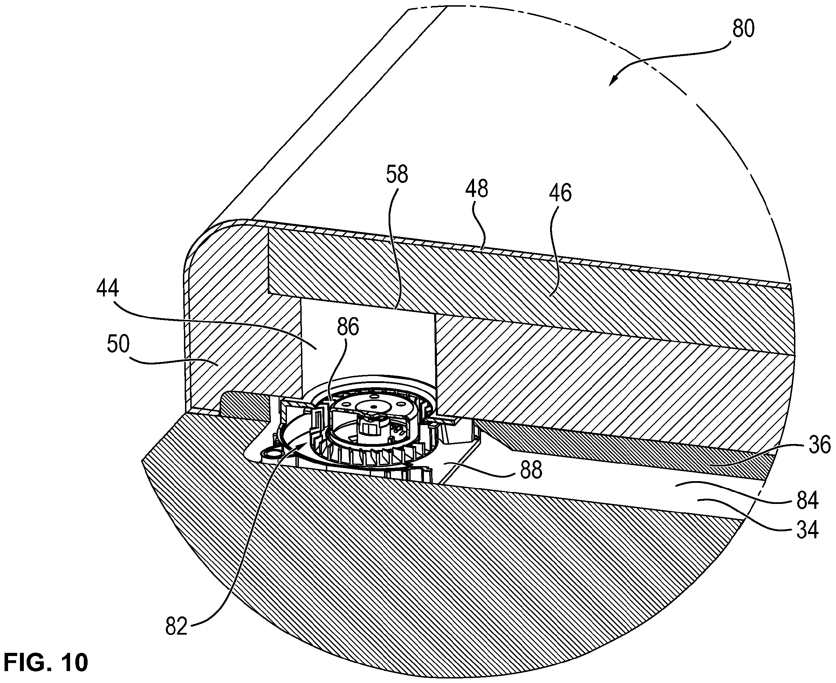

Figur 10 zeigt eine perspektivische Darstellung eines vergrößerten teilgeschnittenen Ausschnitts des Lagerflächensegments 80 in dem die Umwälzeinheit 82 angeordnet ist. Die Umwälzeinheit 82 hat eine Lufteintrittsöffnung 86, durch die Luft aus der Austrittsöffnung 44 angesaugt wird, und eine Austrittsöffnung 88 der Umwälzeinheit 82, durch die der Luftstrom in die Aussparung 34 austritt und im Strömungskanal 34 an dem Flächenheizelement 84 vorbeiströmt und dabei erwärmt wird.

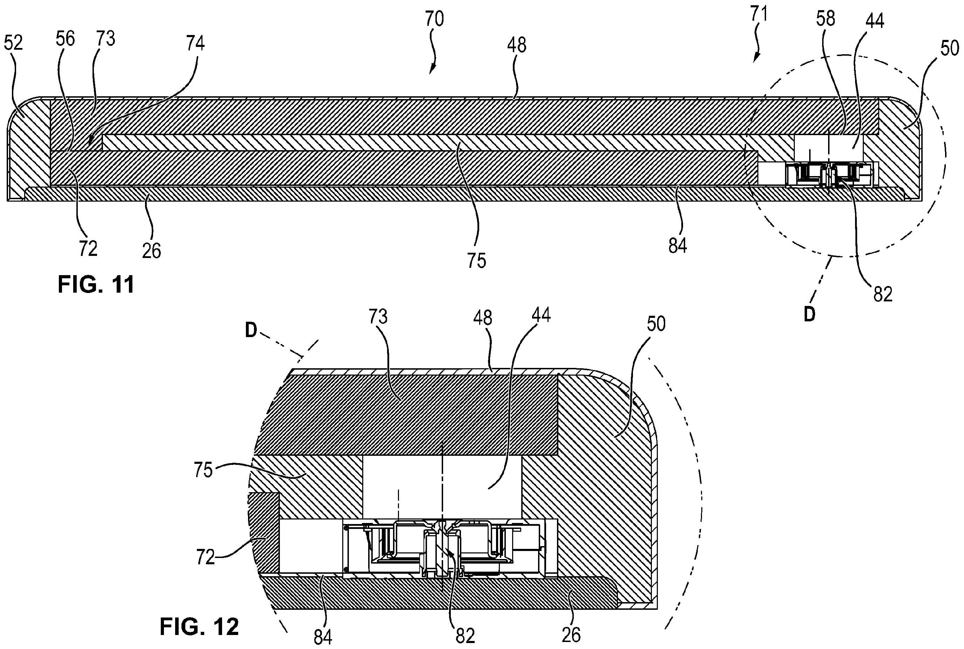

Figur 11 zeigt einen Querschnitt eines Lagerflächensegments 70 einer Vorrichtung 71 zum Erwärmen des Lagerflächensegments 70 gemäß einer dritten Ausführungsform. Die dritte Ausführungsform unterscheidet sich von der zweiten Ausführungsform dadurch, dass der Luftstrom nicht in einem freien Strömungskanal 34 über das Flächenheizelement 84 geführt wird, sondern durch eine über dem Flächenheizelement 84 angeordnete luftdurchlässige weitere Strömungsschicht 72 geführt und darin erwärmt wird. Ferner hat das Lagerflächensegment 70 anstatt der Polsterschicht 38 eine Polsterschicht 75 und anstatt der Strömungsschicht 46 eine Strömungsschicht 73, die einen die weitere Strömungsschicht 72 kontaktierenden Bereich 74 hat. Dieser Bereich 74 ersetzt die Zuleitungsöffnung 42. Dadurch kann die Bauhöhe des Lagerflächensegments 70 und somit des Gesamtsystems reduziert werden.

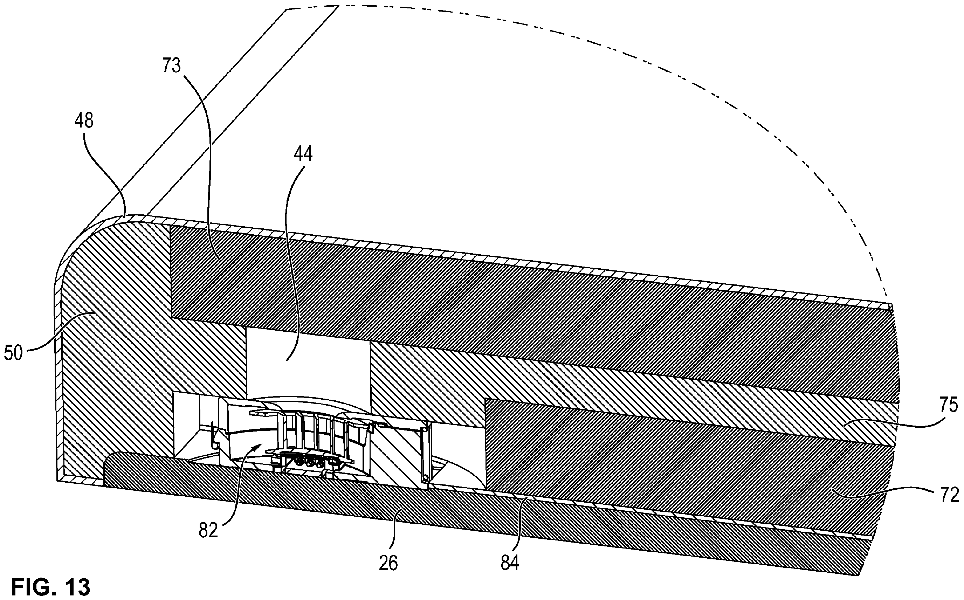

Figur 12 ist eine vergrößerte Darstellung des in Figur 11 gekennzeichneten Bereichs D des Lagerflächensegments 70. In Figur 13 ist eine perspektivische Darstellung eines vergrößerten teilgeschnittenen Ausschnitts des Lagerflächensegments 70 gezeigt. Die Figuren 12 und 13 zeigen, dass der Luftstrom oberhalb der inneren Trägerplatte 36 im Wesentlichen in Materialien mit polsternden Eigenschaften geführt wird.

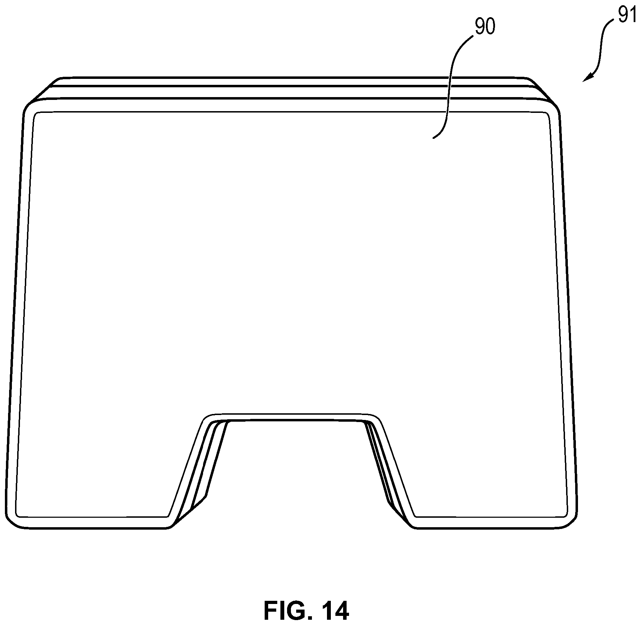

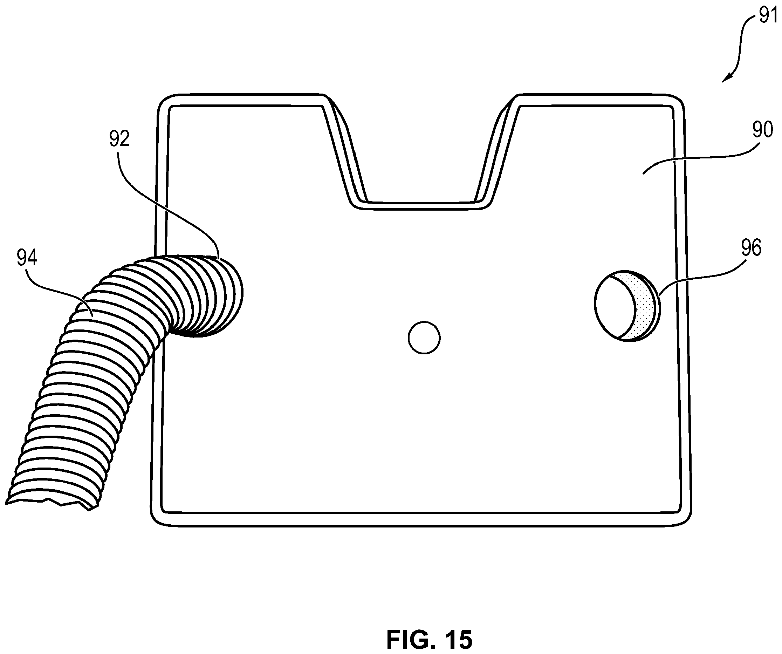

Figur 14 zeigt eine Draufsicht eines Lagerflächensegments 90 einer Vorrichtung 91 gemäß einer vierten Ausführungsform, dessen Unterseite in Figur 15 gezeigt ist. Das Lagerflächensegment 90 hat an seiner Innenseite einen ersten Anschluss 92, an dem ein erster Schlauch 94 angeschlossen ist und einen zweiten Anschluss 96, an dem ein in Figur 15 nicht dargestellter zweiter Schlauch angeschlossen ist.

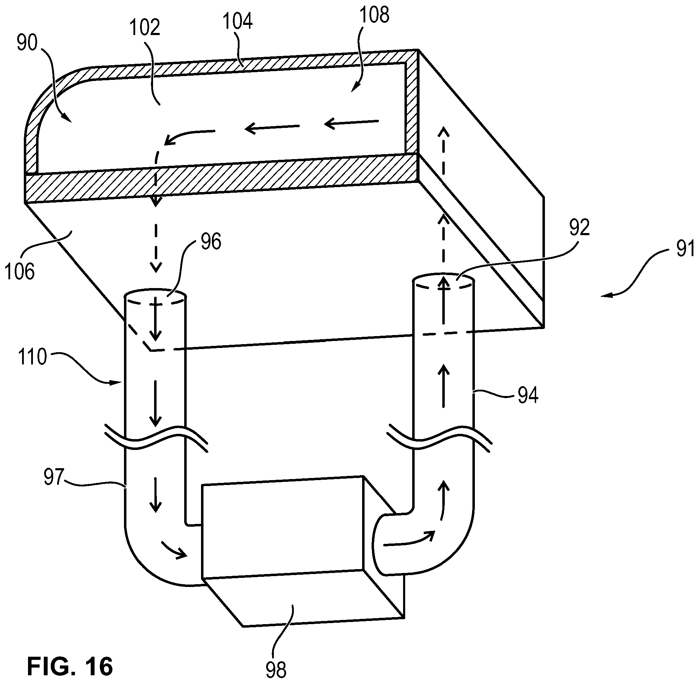

Figur 16 zeigt eine schematische perspektivische Darstellung eines Querschnitts des Lagerflächensegments 90 und einer externen Wärmeerzeugungs- und Umwälzeinheit 98. Die Wärmeerzeugungs- und Umwälzeinheit 98 ist über den ersten Schlauch 94 mit dem ersten Anschluss 92 des Lagerflächensegments 90 und über den zweiten Schlauch 97 mit dem zweiten Anschluss 96 des Lagerflächensegments 90 verbunden.

Das Lagerflächensegment 90 hat eine luftdurchlässige Strömungsschicht 102, eine luftundurchlässige Dichtungsschicht 104 und eine Trägerplatte 106. Die Strömungsschicht 102 und die Dichtungsschicht 104 bilden eine elastisch verformbare Polstereinheit 108. Ferner bilden die Strömungsschicht 102, der erste Anschluss 92, der erste Schlauch 94, der zweite Anschluss 96, der zweite Schlauch 97 und die Wärme- und Umwälzeinheit 98 ein Strömungskanalsystem 110 der Vorrichtung 91, durch das Luft in einem geschlossenen Kreislauf strömt.

Die Strömungsschicht 102 wird von der durch die Wärmeerzeugungs- und Umwälzeinheit 98 erzeugten Strömung in Richtung der eingezeichneten Pfeilkette von erwärmter Luft durchströmt und dabei erwärmt. Die Luft tritt anschließend aus dem zweiten Anschluss 96 des Lagerflächensegments 90 aus und wird durch den zweiten Schlauch 97 der Wärmeerzeugungs- und Umwälzeinheit 98 zugeführt. Die Wärmeerzeugungs- und Umwälzeinheit 98 erwärmt und reinigt die beim Erwärmen der Strömungsschicht 102 erkaltete Luft und führt sie durch den ersten Schlauch 94 über den ersten Anschluss 92 dem Lagerflächensegment 90 wieder zu. Im Übrigen entsprechen Aufbau und Funktion der Vorrichtung 91 der Vorrichtung 23.

Bei einer Vorrichtung gemäß einer nicht dargestellten fünften Ausführungsform strömt die Luft in einem offenen Kreislauf. Dabei saugt die Wärmeerzeugungs- und Umwälzeinheit 98 Luft aus der Umgebung an, erwärmt und reinigt diese und führt sie durch den ersten Schlauch 94 über den ersten Anschluss 92 dem Lagerflächensegment 90 zu. Nach dem Durchströmen der Strömungsschicht 102 tritt die Luft aus dem zweiten Anschluss 96 in die Umgebung aus. Der weitere Aufbau und die Funktion der Vorrichtung gemäß der fünften Ausführungsform entsprechen denen der Vorrichtung 91.

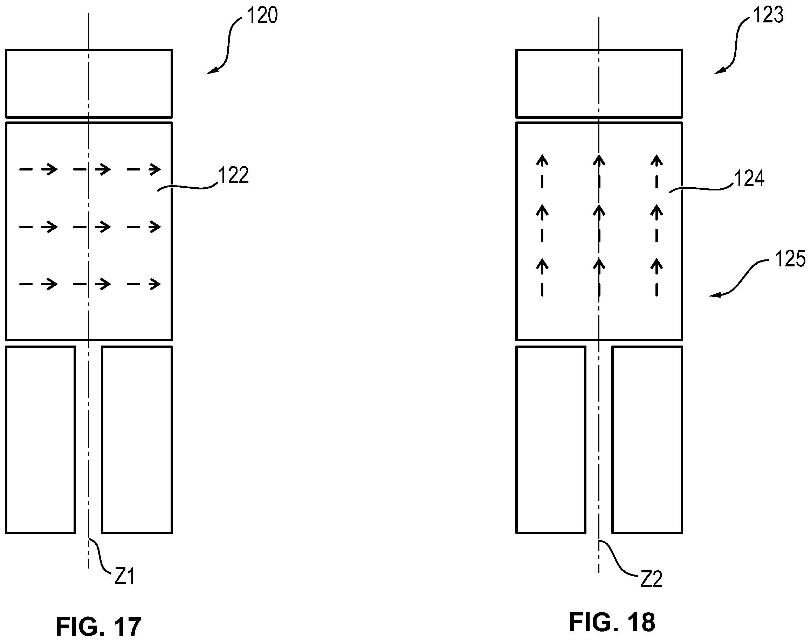

In Figur 17 ist eine Draufsicht einer schematisch dargestellten Patientenlagerfläche 120 gezeigt, die ein quer zur Längsachse Z1 der Patientenlagerfläche 120 durchströmtes Lagerflächensegment 122 hat, das bei vorliegender Ausführungsform als Beckenplatte dient. Im Übrigen entsprechen Aufbau und Funktion des Lagerflächensegments 122 der Beckenplatte 18.

In Figur 18 ist eine Draufsicht einer schematisch dargestellten Patientenlagerfläche 123 gezeigt, die ein von Luft in Richtung der Längsachse Z2 der Patientenlagerfläche 123 durchströmtes Lagerflächensegment 124 einer Vorrichtung 125 zum Erwärmen des Lagerflächensegments 124 hat. Der Eintrittsbereich der Strömungsschicht und der Austrittsbereich der Strömungsschicht sind bei dem Lagerflächensegment 124 der Patientenlagerfläche 123 im Unterschied zu den Lagerflächensegmenten 18, 80 und 90 gemäß den Ausführungsformen eins bis fünf in einem Abstand entlang der Längsachse Z2 angeordnet, sodass das Lagerflächensegment 124 längs mit Luft durchströmt wird. Im Übrigen entsprechen Aufbau und Funktion des Lagerflächensegments 124 dem Lagerflächensegment 18.

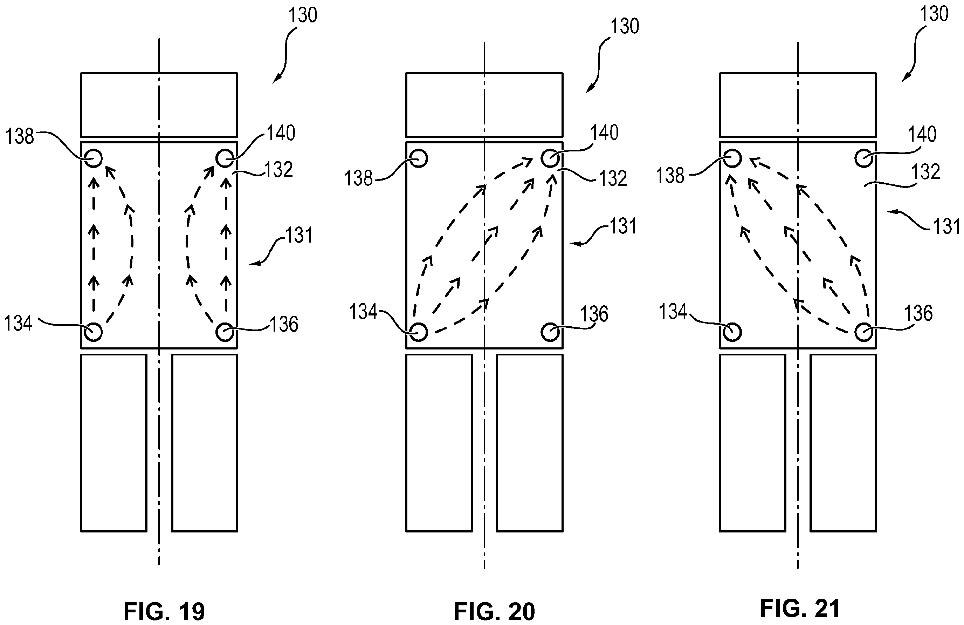

In den Figuren 19, 20 und 21 sind jeweils eine Draufsicht einer schematisch dargestellten Patientenlagerfläche 130 gezeigt, die eine Vorrichtung 131 zum Erwärmen eines Lagerflächensegments 132 gemäß einer sechsten Ausführungsform hat. Die Figuren 16 bis 18 zeigen unterschiedliche Konfigurationen zum Durchströmen des Lagerflächensegments 132 mit Luft. Das Lagerflächensegment 132 hat eine Strömungsschicht mit einem ersten Eintrittsbereich 134 und einem zweiten Eintrittsbereich 136, die jeweils einen Einlass zum Einleiten erwärmter Luft in das Lagerflächensegment 132 haben, einem ersten Austrittsbereich 138 und einem zweiten Austrittsbereich 140, die jeweils einen Auslass zum Weiterleiten der abgekühlten Luft haben. Der weitere Aufbau der Vorrichtung 131 entspricht dem der dritten Ausführungsform. Es sind Ventil- und/oder Klappenanordnungen vorgesehen, um den Eintrittsbereich gezielt erwärmte Luft zuzuführen und gezielt Luft aus dem Austrittsbereich entweichen zu lassen.

Bei der in Figur 19 gezeigten Konfiguration wird der Strömungsschicht durch die Eintrittsbereiche 134 und 136 gleichzeitig erwärmte Luft zugeführt. Die erwärmte Luft durchströmt die Strömungsschicht längs des Lagerflächensegments 132, erwärmt die Strömungsschicht und tritt aus den Austrittbereichen 138 und 140 aus der Strömungsschicht aus.

Der in Figur 20 gezeigten Strömungsschicht wird über den ersten Eintrittsbereich 134 erwärmte Luft zugeführt. Die erwärmte Luft durchströmt die Strömungsschicht des Lagerflächensegments 132 diagonal und tritt durch den zweiten Austrittsbereich 140 aus der Strömungsschicht aus.

In der in Figur 21 gezeigten Konfiguration wird der Strömungsschicht über den zweiten Eintrittsbereich 136 erwärmte Luft zugeführt. Die erwärmte Luft durchströmt die Strömungsschicht des Lagerflächensegments 132 diagonal in Richtung des ersten Austrittsbereichs 138 und tritt durch diesen aus der Strömungsschicht aus. Insbesondere bei den in Figur 20 und Figur 21 gezeigten Konfigurationen ist den Eintrittsbereichen mit Hilfe eines durch eine Steuereinheit angesteuerten Ventilsystems erwärmte Luft zuführbar und die in der jeweiligen Figur gezeigte Durchströmung des Lagerflächensegments 132 erreichbar. Beispielsweise sind die in den Figuren 20 und 21 gezeigten Durchströmungen des Lagerflächensegments in einer Folge abwechselnd nacheinander mit Hilfe der Steuereinheit erzeugbar.

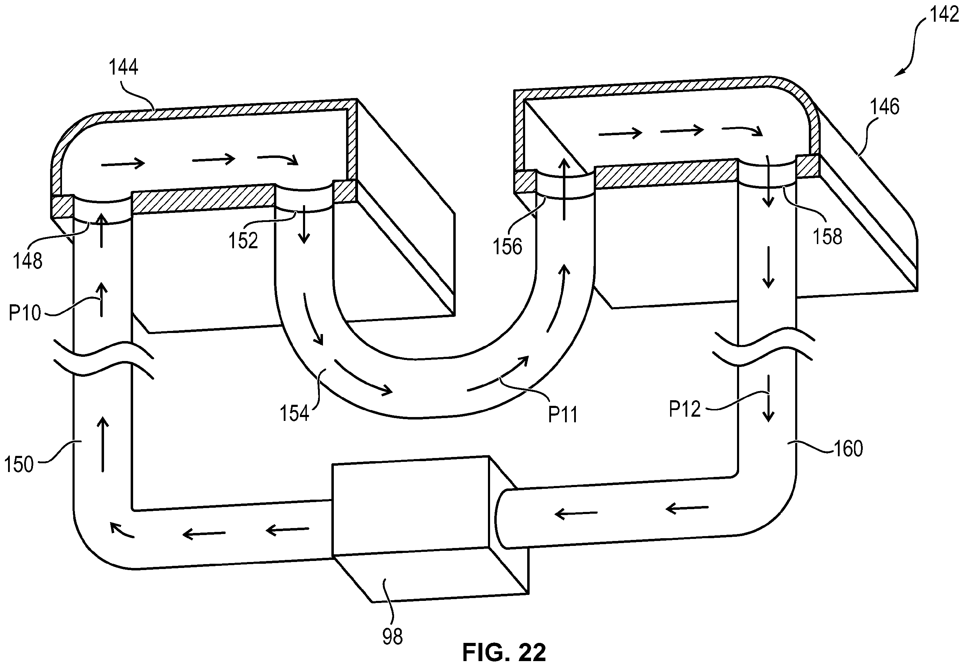

In Figur 22 ist eine schematische perspektivische Ansicht einer Vorrichtung 142 zum Erwärmen von zwei sequentiell von Luft durchströmbaren Lagerflächensegmenten 144, 146 gemäß einer siebten Ausführungsform gezeigt. Das erste Lagerflächensegment 144 hat einen ersten Anschluss 148, mit dem ein erstes Ende einer Luftzuführung 150 verbunden ist, und einen zweiten Anschluss 152, mit dem ein erstes Ende einer Luftverbindung 154 verbunden ist.

Ferner hat das zweite Lagerflächensegment 146 einen ersten Anschluss 156, mit dem das zweite Ende der Luftverbindung 154 verbunden ist, und einen zweiten Anschluss 158, mit dem eine Luftrückführung 160 verbunden ist. Das andere Ende der Luftrückführung 160 und das andere Ende der Luftzuführung 150 sind mit der Wärmeerzeugungs- und Umwälzeinheit 98 verbunden. Der weitere Aufbau der Lagerflächensegmente 144 und 146 entspricht jeweils dem des Lagerflächensegments 90.

Die Wärmeerzeugungs- und Umwälzeinheit 98 erzeugt einen Luftstrom, der durch die Luftzuführung 150 in Richtung des Pfeils P10 strömt und über den ersten Anschluss 148 in die Strömungsschicht des ersten Lagerflächensegments 144 eintritt. Nach dem Durchströmen der Strömungsschicht des ersten Lagerflächensegments 144 tritt die Luft aus dem zweiten Anschluss 152 aus dem ersten Lagerflächensegment 144 aus, strömt durch die Luftverbindung 154 in Richtung des Pfeils P7 zum zweiten Anschluss 156 und gelangt über diesen in die Strömungsschicht des zweiten Lagerflächensegments 146. Nach dem Durchströmen der Strömungsschicht des zweiten Lagerflächensegments 146 tritt die abgekühlte Luft über den zweiten Anschluss 158 aus dem zweiten Lagerflächensegment 146 aus und wird über die Luftrückführung 160 in Richtung des Pfeils P8 der Wärmeerzeugungs- und Umwälzeinheit 98 zugeführt, die die Luft wieder erwärmt.

Anstatt der in Verbindung mit den Figuren beschriebenen Beckenplatten 18 und 122 können die Vorrichtungen 23, 71, 81, 91, 125, 131 und 142 zum Erwärmen in Verbindung mit beliebigen anderen Lagerflächensegmenten genutzt werden. Ferner kann anstatt der durch die Polstereinheiten 24 und 108 geleiteten Luft ein anderes Fluid, insbesondere ein anderes Gas oder Gasgemisch oder eine Flüssigkeit, verwendet werden.

### Bezugszeichenliste

- 10, 120, 130: Patientenlagerfläche
- 12: Kopfplatte
- 14: Rückenplatte
- 16: Rumpfplatte
- 18, 70, 80, 90, 122, 124, 132, 144, 146: Lagerflächensegment
- 20: zweiteilige rechte Beinplatte
- 22: zweiteilige linke Beinplatte
- 23, 71, 81, 91, 125, 131, 142: Vorrichtung
- 24, 108: Polstereinheit
- 26: Trägerplatte
- 30, 98: Wärmeerzeugungs- und Umwälzeinheit
- 32, 110: Strömungskanalsystem
- 34, 45: Aussparungen
- 36: innere Trägerplatte
- 38, 75: Polsterschicht
- 42: Zuleitungsöffnung
- 44: Austrittsöffnung
- 46, 72, 73, 102: Strömungsschicht
- 48, 104: Dichtungsschicht
- 50, 52: Seitenpolster
- 54, 82: Umwälzeinheit
- 56, 134, 136: Eintrittsbereich
- 58, 138, 140: Austrittsbereich
- 60: Heizelement
- 62: Lufteintrittsöffnung
- 64: Luftaustrittsöffnung
- 66: Radialventilator
- 68a bis 68c: Austrittsöffnung
- 74: Bereich
- 84: Flächenheizelement
- 86: Eintrittsöffnung
- 88: Austrittsöffnung
- 92, 96: Anschlüsse
- 94, 100: Schlauch
- 150: Luftzuführung
- 154: Luftverbindung
- 160: Luftrückführung
- P1 bis P12: Pfeile
- A, B, C: Ausschnitt
- Z1, Z2: Längsachse

## Patentansprüche

1. Patientenlagerfläche (10) eines Operationstisches mit einer Vorrichtung (23) zum Erwärmen der Patientenlagerfläche (10),
mit einem Heizelement (60) zum Erwärmen eines zur Wärmeübertragung dienenden Fluids,
einer elastisch verformbaren vom erwärmten Fluid durchströmten Strömungsschicht (46) der Patientenlagerfläche (10), und
mit einer fluidundurchlässigen Dichtungsschicht (48), die auf der Strömungsschicht (46) und einem ersten luftundurchlässigen Seitenpolster (50) und einem zweiten luftundurchlässigen Seitenpolster (52) angeordnet ist, wobei das erste Seitenpolster (50) auf einer Seite neben der Strömungsschicht (46) und das zweite Seitenpolster (52) auf der anderen Seite neben der Strömungsschicht (46) angeordnet ist, wobei eine von der Strömungsschicht (46) abgewandte, äußere Oberfläche der Dichtungsschicht (48) eine zum Patientenkontakt vorgesehene Oberfläche der Patientenlagerfläche (10) ist,
wobei die Strömungsschicht (46) mindestens einen Eintrittsbereich (56) zum Einleiten des von dem Heizelement (60) erwärmten Fluids in die Strömungsschicht (46) hat, und
wobei die Strömungsschicht (46) mindestens einen Austrittsbereich (58) zum Austreten des Fluids aus der Strömungsschicht (46) hat, und wobei der Eintrittsbereich (56) an das zweite Seitenpolster (52) angrenzt und wobei der Austrittsbereich (58) an das erste Seitenpolster (52) angrenzt.

2. Patientenlagerfläche (10) nach Anspruch 1, **dadurch gekennzeichnet, dass** ein Strömungserzeuger (54) vorgesehen ist, mit dessen Hilfe ein Fluidstrom zum Einleiten des Fluids in den Eintrittsbereich (56) der Strömungsschicht (46), zum Durchströmen der Strömungsschicht (46) und zum Austreten aus dem Austrittsbereich (58) der Strömungsschicht (46) erzeugbar ist.

3. Patientenlagerfläche (10) nach einem der vorhergehenden Ansprüche, **dadurch gekennzeichnet, dass** die Patientenlagerfläche (10) mindestens ein Lagerflächensegment (18) mit einem Polster (24) hat,
dass die Oberfläche der Patientenlagerfläche (10) eine Oberfläche des Polsters (24) des Lagerflächensegments (18) ist, und
dass die Strömungsschicht (46) und die Dichtungsschicht (48) in dem Polster (24) des Lagerflächensegments (18) angeordnet sind.

4. Patientenlagerfläche (10) nach einem der vorhergehenden Ansprüche, **dadurch gekennzeichnet, dass** die Strömungsschicht (46) durch einen offenporigen Schaumstoff gebildet ist.

5. Patientenlagerfläche (10) nach einem der Ansprüche 2 bis 4, **dadurch gekennzeichnet, dass** die Patientenlagerfläche (10) in den durch den Auflagedruck eines auf der Patientenliegefläche (10) befindlichen Patienten stärker verformten Bereichen der Strömungsschicht (46) bedingt durch den im Vergleich zu den übrigen Bereichen der Strömungsschicht (46) reduzierten Volumenstrom des Fluids weniger erwärmt ist.

6. Patientenlagerfläche (10) nach einem der vorhergehenden Ansprüche, **dadurch gekennzeichnet, dass** eine an die Strömungsschicht (46) angrenzende Wärmespeicherschicht zum Speichern der Wärme des Fluids vorgesehen ist.

7. Patientenlagerfläche (10) nach einem der Ansprüche 1 bis 6, **dadurch gekennzeichnet, dass** der mindestens eine Eintrittsbereich (56) und der mindestens eine Austrittsbereich (58) außerhalb eines für die Durchleuchtung des Patienten mit bildgebenden Verfahren vorgesehenen Bereichs der Patientenlagerfläche (10) angeordnet sind.

8. Patientenlagerfläche (10) nach einem der vorhergehenden Ansprüche, **dadurch gekennzeichnet, dass** das Heizelement (60) in der Patientenlagerfläche (10) angeordnet ist.

9. Patientenlagerfläche (10) nach Anspruch 8, **dadurch gekennzeichnet, dass** das Heizelement ein röntgentransparentes Flächenheizelement (84) ist, und
dass das Fluid bei aktiviertem Strömungserzeuger (54) an der Heizfläche des Flächenheizelements (84) vorbeiströmt.

10. Patientenlagerfläche (123) nach einem der Ansprüche 3 bis 9, **dadurch gekennzeichnet, dass** der Eintrittsbereich (56) an einem ersten Ende des Lagerflächensegments (124) und der Austrittsbereich (58) an einem dem ersten Ende in Längsrichtung (Z2) der Patientenlagerfläche (123) gegenüberliegenden zweiten Ende des Lagerflächensegments (124) angeordnet sind.

11. Patientenlagerfläche (130) nach einem der Ansprüche 3 bis 10, **dadurch gekennzeichnet, dass** die Strömungsschicht (102) des Lagerflächensegments (132) einen weiteren Eintrittsbereich (136) zum Einleiten des von dem Heizelement (98) erwärmten Fluids in die Strömungsschicht (102) hat, und/oder
dass die Strömungsschicht (102) das Lagerflächensegment (132) einen weiteren Austrittsbereich (140) zum Austreten des Fluids aus der Strömungsschicht (102) hat.

12. Patientenlagerfläche (130) nach Anspruch 11, **dadurch gekennzeichnet, dass** eine Steuereinheit und ein Ventilsystem zum Steuern des Fluidstroms durch die Strömungsschicht (102) vorgesehen sind, und
dass das Ventilsystem derart durch die Steuereinheit steuerbar ist, dass das Fluid nur in einem Eintrittsbereich (134, 136) in die Strömungsschicht (102) eingeleitet wird.

13. Patientenlagerfläche (10) nach einem der vorhergehenden Ansprüche, **gekennzeichnet durch** eine Reinigungseinheit zum Reinigen von kontaminiertem Fluid.

14. Patientenlagerfläche (10) nach einem der Ansprüche 3 bis 13, **dadurch gekennzeichnet, dass** das Lagerflächensegment ein erstes Lagerflächensegment (144) ist,
dass das erste Lagerflächensegment (144) einen ersten Anschluss (148) zum Einleiten des Fluids in das erste Lagerflächensegment (144) und einen zweiten Anschluss (152) zum Austreten des Fluids aus dem ersten Lagerflächensegment (144) hat,
dass die Patientenlagerfläche mindestens ein zweites Lagerflächensegment (146) mit einem ersten Anschluss (156) zum Einleiten des Fluids in das zweite Lagerflächensegment (146) und einen zweiten Anschluss (158) zum Austreten des Fluids aus dem zweiten Lagerflächensegment (146) hat,
dass das zweite Lagerflächensegment (146) eine elastisch verformbare vom erwärmten Fluid durchströmbare Strömungsschicht und eine zwischen einer zum Patientenkontakt vorgesehenen Oberfläche des zweiten Lagerflächensegments (146) und der Strömungsschicht des zweiten Lagerflächensegments (146) angeordnete fluidundurchlässige Dichtungsschicht hat,
dass die Strömungsschicht des zweiten Lagerflächensegments (146) mindestens einen Eintrittsbereich zum Einleiten des von dem Heizelement (98) erwärmten Fluids in die Strömungsschicht des zweiten Lagerflächensegments (146) und mindestens einen Austrittsbereich zum Austreten des Fluids aus der Strömungsschicht des zweiten Lagerflächensegments (146) hat, und
dass der zweite Anschluss (152) des ersten Lagerflächensegments (144) mit dem ersten Anschluss (156) des zweiten Lagerflächensegments (146) derart verbunden ist, dass das Fluid bei aktiviertem Strömungserzeuger (98) durch den ersten Anschluss (148) des ersten Lagerflächensegments (144) in das erste Lagerflächensegment (144) eingeleitet wird, von dem ersten Lagerflächensegment (144) in das zweite Lagerflächensegment (146) strömt, in den Eintrittsbereich der Strömungsschicht des zweiten Lagerflächensegments (146) eingeleitet wird, aus dem Austrittsbereich der Strömungsschicht des zweiten Lagerflächensegments (146) austritt und aus dem zweiten Anschluss (158) des zweiten Lagerflächensegments (146) austritt.

15. Patientenlagerfläche (10) nach einem der Ansprüche 2 bis 14, **gekennzeichnet durch** einen geschlossenen Fluidkreislauf, in dem die Strömungsschicht (46), das Heizelement (60) und der Strömungserzeuger (54) angeordnet sind.

16. Patientenlagerfläche (10) nach einem der Ansprüche 2 bis 14, **dadurch gekennzeichnet, dass** Luft als Fluid vorgesehen ist,
dass Luft aus der Umgebung mit Hilfe des Strömungserzeugers (98) ansaugbar ist, und
dass die bei aktiviertem Strömungserzeuger (98) angesaugte Luft aus der Umgebung in den Eintrittsbereich der Strömungsschicht (102) des Lagerflächensegments (90) eingeleitet wird.

## Claims

1. A patient bearing area (10) of an operating table having a device (23) for heating the patient bearing area (10),
having a heating element (60) for heating a fluid used for heat transfer,
an elastically deformable flow layer (46) of the patient bearing area (10) through which the heated fluid flows, and
having a fluid-impermeable sealing layer (48) which is arranged on the flow layer (46) and a first air-impermeable side cushion (50) and a second air-impermeable side cushion (52), wherein the first side cushion (50) is arranged on one side next to the flow layer (46) and the second side cushion (52) is arranged on the other side next to the flow layer (46), wherein an outer surface of the sealing layer (48) facing away from the flow layer (46) is a surface of the patient bearing area (10) intended for patient contact,
wherein the flow layer (46) has at least one inlet region (56) for introducing the fluid heated by the heating element (60) into the flow layer (46), and
wherein the flow layer (46) has at least one outlet region (58) for exiting the fluid from the flow layer (46), and wherein the inlet region (56) adjoins the second side cushion (52) and wherein the outlet region (58) adjoins the first side cushion (52) .

2. The patient bearing area (10) according to claim 1, **characterized in that** a flow generator (54) is provided, with the aid of which a fluid flow can be generated for introducing the fluid into the inlet region (56) of the flow layer (46), for flowing through the flow layer (46), and for exiting from the outlet region (58) of the flow layer (46).

3. The patient bearing area (10) according to any one of the preceding claims, **characterized in that** the patient bearing area (10) has at least one bearing area segment (18) having a cushion (24),
**in that** the surface of the patient bearing area (10) is a surface of the cushion (24) of the bearing area segment (18), and
**in that** the flow layer (46) and the sealing layer (48) are arranged in the cushion (24) of the bearing surface segment (18) .

4. The patient bearing area (10) according to any one of the preceding claims, **characterized in that** the flow layer (46) is formed by an open-pore foam.

5. The patient bearing area (10) according to any one of claims 2 to 4, **characterized in that** the patient bearing area (10) is heated less in the regions of the flow layer (46) which are more strongly deformed by the contact pressure of a patient on the patient bearing area (10) due to the reduced volume flow of the fluid in comparison to the remaining regions of the flow layer (46) .

6. The patient bearing area (10) according to any one of the preceding claims, **characterized in that** a heat storage layer adjacent to the flow layer (46) is provided for storing the heat of the fluid.

7. The patient bearing area (10) according to any one of claims 1 to 6, **characterized in that** the at least one inlet region (56) and the at least one outlet region (58) are arranged outside a region of the patient bearing area (10) intended for fluoroscopy of the patient using imaging methods.

8. The patient bearing area (10) according to any one of the preceding claims, **characterized in that** the heating element (60) is arranged in the patient bearing area (10).

9. The patient bearing area (10) according to claim 8, **characterized in that** the heating element is an X-ray-transparent surface heating element (84), and
**in that** the fluid flows past the heating surface of the surface heating element (84) when the flow generator (54) is activated.

10. The patient bearing area (123) according to any one of claims 3 to 9, **characterized in that** the inlet region (56) is arranged at a first end of the bearing area segment (124) and the outlet region (58) is arranged at a second end of the bearing surface segment (124) opposite to the first end in the longitudinal direction (Z2) of the patient bearing area (123).

11. The patient bearing area (130) according to any one of claims 3 to 10, **characterized in that** the flow layer (102) of the bearing area segment (132) has a further inlet region (136) for the introduction of the fluid heated by the heating element (98) into the flow layer (102), and/or
**in that** the flow layer (102) of the bearing surface segment (132) has a further outlet region (140) for exiting the fluid from the flow layer (102).

12. The patient bearing area (130) according to claim 11, **characterized in that** a control unit and a valve system are provided for controlling the fluid flow through the flow layer (102), and
**in that** the valve system is controllable by the control unit in such a way that the fluid is introduced into the flow layer (102) only in an inlet region (134, 136).

13. The patient bearing area (10) according to any one of the preceding claims, **characterized by** a purification unit for purifying contaminated fluid.

14. The patient bearing area (10) according to any one of claims 3 to 13, **characterized in that** the bearing area segment is a first bearing area segment (144),
**in that** the first bearing surface segment (144) has a first connection (148) for the introduction of the fluid into the first bearing surface segment (144) and a second connection (152) for the exit of the fluid from the first bearing surface segment (144),
**in that** the patient bearing surface has at least one second bearing surface segment (146) having a first connection (156) for introducing the fluid into the second bearing surface segment (146) and a second connection (158) for exiting the fluid from the second bearing surface segment (146),
**in that** the second bearing surface segment (146) has an elastically deformable flow layer through which the heated fluid can flow and a fluid-impermeable sealing layer arranged between a surface of the second bearing surface segment (146) intended for patient contact and the flow layer of the second bearing surface segment (146),
**in that** the flow layer of the second bearing surface segment (146) has at least one inlet region for introducing the fluid heated by the heating element (98) into the flow layer of the second bearing surface segment (146) and at least one outlet region for exiting the fluid from the flow layer of the second bearing surface segment (146), and
**in that** the second connection (152) of the first bearing surface segment (144) is connected to the first connection (156) of the second bearing surface segment (146) in such a way that when the flow generator (98) is activated, the fluid is introduced through the first connection (148) of the first bearing surface segment (144) into the first bearing surface segment (144), flows from the first bearing surface segment (144) into the second bearing surface segment (146), is introduced into the inlet region of the flow layer of the second bearing surface segment (146), exits from the outlet region of the flow layer of the second bearing surface segment (146), and exits from the second connection (158) of the second bearing surface segment (146) .

15. The patient bearing area (10) according to any one of claims 2 to 14, **characterized by** a closed fluid circuit in which the flow layer (46), the heating element (60), and the flow generator (54) are arranged.

16. The patient bearing area (10) according to any one of claims 2 to 14, **characterized in that** air is provided as the fluid,
**in that** air can be aspirated from the environment with the aid of the flow generator (98), and
**in that** the air aspirated from the environment - when the flow generator (98) is activated - is introduced into the inlet region of the flow layer (102) of the bearing area segment (90).

## Revendications

1. Surface de réception de patient (10) d'une table d'opération dotée d'un dispositif (23) pour chauffer la surface de réception de patient (10),
dotée d'un élément chauffant (60) pour chauffer un fluide utilisé pour le transfert de chaleur,
d'une couche d'écoulement (46) de la surface de support du patient (10), déformable élastiquement, à travers laquelle s'écoule le fluide chauffé, et
dotée d'une couche d'étanchéité imperméable aux fluides (48) qui est disposée sur la couche d'écoulement (46) et sur un premier coussin latéral imperméable à l'air (50) et sur un second coussin latéral imperméable à l'air (52), dans laquelle le premier coussin latéral (50) est disposé sur un côté à côté de la couche d'écoulement (46) et le second coussin latéral (52) est disposé sur l'autre côté à côté de la couche d'écoulement (46), dans laquelle une surface externe de la couche d'étanchéité (48) opposée à la couche d'écoulement (46) est une surface de la surface de réception de patient (10) destinée au contact avec le patient,
dans laquelle la couche d'écoulement (46) présente au moins une zone d'entrée (56) pour introduire le fluide chauffé par l'élément chauffant (60) dans la couche d'écoulement (46), et
dans laquelle la couche d'écoulement (46) présente au moins une zone de sortie (58) pour faire sortir le fluide de la couche d'écoulement (46), et dans laquelle la zone d'entrée (56) est adjacente au second coussin latéral (52) et dans laquelle la zone de sortie (58) est adjacente au premier coussin latéral (52) .

2. Surface de réception de patient (10) selon la revendication 1, **caractérisée en ce qu'**un générateur de flux (54) est prévu, à l'aide duquel un écoulement de fluide peut être créé pour introduire le fluide dans la zone d'entrée (56) de la couche d'écoulement (46), pour le faire s'écouler à travers la couche d'écoulement (46) et pour le faire sortir de la zone de sortie (58) de la couche d'écoulement (46).

3. Surface de réception de patient (10) selon l'une des revendications précédentes, **caractérisée en ce que** la surface de réception de patient (10) présente au moins un segment de surface de réception (18) avec un coussin (24),
**en ce que** la surface de la surface de réception de patient (10) est une surface du coussin (24) du segment de surface de réception (18), et
**en ce que** la couche d'écoulement (46) et la couche d'étanchéité (48) sont disposées dans le coussin (24) du segment de surface de réception (18).

4. Surface de réception de patient (10) selon l'une des revendications précédentes, **caractérisée en ce que** la couche d'écoulement (46) est formée d'une mousse à pores ouverts.

5. Surface de réception de patient (10) selon l'une des revendications 2 à 4, **caractérisée en ce que** la surface de réception de patient (10) est moins chauffée dans les zones de la couche d'écoulement (46) qui sont plus fortement déformées par la pression de contact d'un patient se trouvant sur la surface de réception de patient (10), en raison du débit volumique réduit du fluide par rapport aux zones restantes de la couche d'écoulement (46).

6. Surface de réception de patient (10) selon l'une des revendications précédentes, **caractérisée en ce qu'**une couche d'accumulation de chaleur adjacente à la couche d'écoulement (46) est prévue pour stocker la chaleur du fluide.

7. Surface de réception de patient (10) selon l'une des revendications 1 à 6, **caractérisée en ce que** l'au moins une zone d'entrée (56) et l'au moins une zone de sortie (58) sont disposées à l'extérieur d'une zone de la surface de support de patient (10) prévue pour la fluoroscopie du patient à l'aide de procédés d'imagerie.

8. Surface de réception de patient (10) selon l'une des revendications précédentes, **caractérisée en ce que** l'élément chauffant (60) est disposé dans la surface de réception de patient (10).

9. Surface de réception de patient (10) selon la revendication 8, **caractérisée en ce que** l'élément chauffant est un élément chauffant de surface transparent aux rayons X (84), et
**en ce que** le fluide s'écoule devant la surface chauffante de l'élément chauffant de surface (84) lorsque le générateur de flux (54) est activé.

10. Surface de réception de patient (123) selon l'une des revendications 3 à 9, **caractérisée en ce que** la zone d'entrée (56) est disposée à une première extrémité du segment de surface de réception (124) et la zone de sortie (58) est disposée à l'une des premières extrémités dans la direction longitudinale (Z2) de la surface de réception de patient (123) opposée à la seconde extrémité du segment de surface de réception (124).

11. Surface de réception de patient (130) selon l'une des revendications 3 à 10, **caractérisée en ce que** la couche d'écoulement (102) du segment de surface de réception (132) présente une autre zone d'entrée (136) pour introduire du fluide chauffé par l'élément chauffant (98) dans la couche d'écoulement (102), et/ou
**en ce que** la couche d'écoulement (102) du segment de surface de réception (132) présente une autre zone de sortie (140) pour faire sortir le fluide de la couche d'écoulement (102).

12. Surface de réception de patient (130) selon la revendication 11, **caractérisée en ce qu'**une unité de commande et un système de soupape sont prévus pour commander l'écoulement de fluide à travers la couche d'écoulement (102), et
**en ce que** le système de soupape peut être commandé par l'unité de commande de sorte que le fluide soit introduit dans la couche d'écoulement (102) uniquement dans une zone d'entrée (134, 136).

13. Surface de réception de patient (10) selon l'une des revendications précédentes, **caractérisée par** une unité de nettoyage pour nettoyer un fluide contaminé.

14. Surface de réception de patient (10) selon l'une des revendications 3 à 13, **caractérisée en ce que** le segment de surface de réception est un premier segment de surface de réception (144),
**en ce que** le premier segment de surface de réception (144) présente un premier raccord (148) pour introduire le fluide dans le premier segment de surface de réception (144) et un second raccord (152) pour faire sortir le fluide du premier segment de surface de réception (144),
**en ce que** la surface de réception du patient présente au moins un second segment de surface de réception (146) avec un premier raccord (156) pour introduire le fluide dans le second segment de surface de réception (146) et un second raccord (158) pour faire sortir le fluide du second segment de surface de réception (146),
**en ce que** le second segment de surface de réception (146) présente une couche d'écoulement élastiquement déformable à travers laquelle le fluide chauffé peut s'écouler et une couche d'étanchéité imperméable aux fluides disposée entre une surface du second segment de surface de réception (146) destinée au contact avec le patient et la couche d'écoulement du second segment de surface de réception (146),
**en ce que** la couche d'écoulement du second segment de surface de réception (146) présente au moins une zone d'entrée pour introduire le fluide chauffé par l'élément chauffant (98) dans la couche d'écoulement du second segment de surface de réception (146) et au moins une zone de sortie pour faire sortir le fluide de la couche d'écoulement du second segment de surface de réception (146), et
**en ce que** le second raccord (152) du premier segment de surface de réception (144) est relié au premier raccord (156) du second segment de surface de réception (146) de sorte que lorsque le générateur de flux (98) est activé, le fluide est introduit à travers le premier raccord (148) du premier segment de surface de réception (144) dans le premier segment de surface de réception (144), s'écoule du premier segment de surface de réception (144) dans le second segment de surface de réception (146), est introduit dans la zone d'entrée de la couche d'écoulement du second segment de surface de réception (146), sort de la zone de sortie de la couche d'écoulement du second segment de surface de réception (146) et sort du second raccord (158) du second segment de surface de réception (146).

15. Surface de réception de patient (10) selon l'une des revendications 2 à 14, **caractérisée par** un circuit fluidique fermé dans lequel sont disposés la couche d'écoulement (46), l'élément chauffant (60) et le générateur de flux (54).

16. Surface de réception de patient (10) selon l'une des revendications 2 à 14, **caractérisée en ce que** de l'air est prévu comme fluide,
**en ce que** l'air ambiant peut être aspiré à l'aide du générateur de flux (98), et
**en ce que** l'air ambiant aspiré lorsque le générateur de flux (98) est activé est introduit dans la zone d'entrée de la couche d'écoulement (102) du segment de surface de réception (90).
